# EUROPÄISCHE PATENTSCHRIFT

(11) **EP 1 077 717 B1**
(45) Veröffentlichungstag und Bekanntmachung des Hinweises auf die Patenterteilung: **23.07.2003**
(21) Anmeldenummer: 99952073.7
(22) Anmeldetag: 19.05.1999
(51) Int. Cl.: A61K 38/03, A61K 38/10, A61K 9/00, A61K 35/74, A61K 9/127, A61P 17/02

(54) **VERWENDUNG VON LIPOPEPTIDEN ODER LIPOPROTEINEN ZUR WUNDBEHANDLUNG**
USE OF LIPOPEPTIDES OR LIPOPROTEINS FOR WOUND TREATMENT
UTILISATION DE LIPOPEPTIDES OU DE LIPOPROTEINES POUR LE TRAITEMENT DE PLAIES

(30) Priorität: 20.05.1998 DE 19822820
(43) Veröffentlichungstag der Anmeldung: 28.02.2001
(73) Patentinhaber: Gesellschaft für Biotechnologische Forschung mbH (GBF), 38124 Braunschweig (DE)
(72) Erfinder: MÜHLRADT, Peter, D-38124 Braunschweig (DE); DEITERS, Ursula, D-38124 Braunschweig (DE)
(74) Vertreter: Boeters, Hans Dietrich, Dr.
(86) Internationale Anmeldenummer: EP9903436
(87) Internationale Veröffentlichungsnummer: WO99059610

(56) Entgegenhaltungen:
- WO-A-89/05653
- P.F. MÜHLRADT ET AL.: "ISOLATION, STRUCTURE ELUCIDATION, AND SYNTHESIS OF A MACROPHAGE STIMULATORY LIPOPEPTIDE FROM MYCOPLASMA FERMENTANS ACTING AT PICOMOLAR CONCENTRATION." JOURNAL OF EXPERIMENTAL MEDICINE, Bd. 185, Nr. 11, 2. Juni 1997 (1997-06-02), Seiten 1951-1958, XP002123770 NEW YORK, N.Y., US in der Anmeldung erwähnt
- P.F. MÜHLRADT ET AL.: "MDHM, A MACROPHAGE-STIMULATORY PRODUCT OF MYCOPLASMA FERMENTANS, LEADS TO IN VITRO INTERLEUKIN-1 (IL-1), IL-6, TUMOR NECROSIS FACTOR, AND PROSTAGLANDIN PRODUCTION AND IS PYROGENIC IN RABBITS" INFECTION AND IMMUNITY, Bd. 59, Nr. 11, November 1991 (1991-11), Seiten 3969-3974, XP002123771 WASHINGTON, US in der Anmeldung erwähnt
- L.A. DIPIETRO: "MIP-1 ALPHA AS A CRITICAL MACROPHAGE CHEMOATTRACTANT IN MURINE WOUND REPAIR." THE JOURNAL OF CLINICAL INVESTIGATION, Bd. 101, Nr. 8, April 1998 (1998-04), Seiten 1693-1698, XP002123772 NEW YORK, N.Y., US

## Beschreibung

### Stand der Technik

Man kann 5 Stadien der Wundheilung beschreiben:
1. Blutgerinnung und Freisetzung von Mediatoren aus Thrombozyten (nach einigen Minuten),
2. Einstrom von Leukozyten, d. h. anfangs Granulozyten, später Makrophagen und Lymphozyten (Tag 1-3),
3. Vermehrung diverser Zellen wie Fibroblasten, Endo- und Epithelzellen (Tag 3-7),
4. Wundkontraktion (Tag 7-9) und
5. Umbau des Narbengewebes (bis zu einem Jahr)

Das Einströmen von Granulozyten in Phase 2 bewirkt Aufnahme von Debris und Abtöten von infektiösen Erregern, eine Aufgabe, die auch von Makrophagen übernommen wird. Die Makrophagen sind darüberhinaus die Quelle einer Reihe von Mediatoren wie Signalpeptiden, Wachstumsfaktoren und Zytokinen, wie Transforming Growth Factor (TGFβ1), Platelet Derived Growth Factor (PDGF-AA und - BB), Fibroblast Growth Factor (FGF2) und zur Familie der Epidermal Growth Factors (EGF) gehörigem TGF-α. Makrophagen sezernieren auch Interleukin-1 (IL-1), das indirekt in Fibroblasten FGF7 induziert. Alle diese Faktoren sind an unterschiedlichen Stadien der Wundheilung beteiligt bzw. dafür unerläßlich. Ohne Makrophagen als Quelle dafür ist eine Wundheilung stark verzögert bzw. nicht möglich.

### Problematik:

Obwohl es möglich ist, einige der oben erwähnten Mediatoren in isolierter Form bei der Wundheilung einzusetzen, ist dies jedoch schwierig, da die meisten dieser Peptide eine Halbwertszeit von nur wenigen Minuten haben. Weitere Schwierigkeiten bestehen darin, daß der natürliche Zeitpunkt des Erscheinens der unterschiedlichen Mediatoren, die optimale Dosierung und die Interaktion dieser Substanzen nicht im Einzelnen bekannt sind, geschweige denn bei Applikation kontrolliert werden können.

Eine Komplikation auch chirurgischer Wunden können Infektionen darstellen, die im allgemeinen zu verzögerter Wundheilung und erhöhter Narbenbildung führen, was besonders bei kosmetischen Operationen problematisch ist. Eine prophylaktische Abdeckung mit Antibiotika ist angesichts der Resistenzprobleme und eventueller allergischer Reaktionen vielerorts nicht mehr üblich. Bei bestimmten Patientengruppen, z. B. Diabetikern oder älteren Patienten, ist Wundheilung verzögert.

### Lösung

Mykoplasmen haben natürlicherweise die Eigenschaft, am Infektionsort, z. B. der Lunge, zum Einströmen von Leukozyten zu führen. Wie wir jetzt zeigen konnten, ist diese Eigenschaft mit dem Vorhandensein einer bestimmten Klasse von Lipopeptiden assoziiert, die dadurch charakterisiert ist, daß sie N-terminal eine Dihydroxypropyl-cystein-Gruppe mit zwei esterartig gebundenen, langkettigen Fettsäuren aufweist (Mühlradt et al. in J. Exp. Med., 185 (1997) 1951). Derartige Lipopeptide haben außer der bereits beschriebenen Eigenschaft, Makrophagen in vitro zur Freisetzung von Zytokinen und Prostaglandinen zu stimulieren (1 bis 3), auch die von uns neu gefundene Fähigkeit, in vivo hohe Titer an den Chemokinen MIP-1α, MIP-2, MCP-1 und KC zu induzieren.

In der Tier- wie Humanmedizin können reine, synthetisch hergestellte Lipopeptide sowie Lipopeptide, die in Liposomen eingebaut oder an biologisch abbaubare polymere Trägersubstanzen gekoppelt sind, z. B. in Form von Salben, Lotionen oder Injektionslösungen eingesetzt werden. Auf Wunden aufgebracht oder in Wundnähe injiziert sollen derartige Präparate den natürlichen Einstrom von Granulozyten und Makrophagen erhöhen und beschleunigen und somit einer Infektion vorbeugen sowie durch Anregung der Biosynthese an der Wundheilung beteiligter Mediatoren in der natürlichen Reihenfolge und Konzentration die Wundheilung erleichtern und beschleunigen. Die in vivo-Wirksamkeit derartiger Lipopeptide aus Mykoplasmen ist überraschend und neu, da die Applikation von anderen bakteriellen Lipopeptiden und ihrer synthetischen Analoga im Tierversuch keine Wirkung zeigen (S. Hausschildt et al. in FEMS Immunol. Med. Microbiol., 8 (1994) 77).

Gemäß einer Ausführungsform wird die der Erfindung zugrundeliegende Aufgabe durch die Verwendung eines physiologisch verträglichen Lipopeptids oder Lipoproteins, das N-terminal eine Dihydroxypropyl-cystein-Gruppe mit zwei esterartig gebundenen gegebenenfalls langkettigen Fettsäuren trägt, die gleich oder verschieden sind, zur Herstellung eines pharmazeutischen Präparats zur Tier- und Humanwundbehandlung gelöst.

Ferner betrifft die Erfindung die Verwendung eines Lipopeptids oder Lipoproteins, erhältlich aus einem Mykoplasma-Klon, zur Herstellung eines pharmazeutischen Präparats zur Tier- oder Humanwundbehandlung.

Diese Verwendung kann dadurch gekennzeichnet sein, daß das Lipopeptid oder Lipoprotein aus einem Mykoplasma fermentans-Klon erhältlich ist.

Bei der erfindungsgemäßen Verwendung kann das Lipopeptid oder Lipoprotein wasserlöslich oder amphoter sein.

Erfindungsgemäß kann S-[2,3-Bispalmitoyloxy-(2RS)-propyl]cysteinyl-GNNDESNISFKEK verwendet werden oder S-[2,3-Bispalmitoyloxy-(2R)-propyl]cysteinyl-GNNDESNISFKEK oder S-[2,3-Bispalmitoyloxy-(2S)-propyl]cysteinyl-GNNDESNISFKEK 03-09-2002.

Bei der erfindungsgemäßen Verwendung kann das Lipopeptid oder Lipoprotein in Form einer Lösung zur epikutanen Anwendung, einer Injektionslösung, einer Salbe, einer Lotion, einer wäßrigen Suspension, eines damit imprägnierten oder beschichteten Pflasters, in Liposomen verkapselt oder an biologisch abbaubare Trägerpolymere gekoppelt vorliegen.

Bei der erfindungsgemäßen Verwendung kann es sich bei den Wunden um Wunden nach Verletzungen oder chirurgischen Eingriffen, um chronisch infizierte Wunden, Brandwunden, chronische Ulcera oder Ulcus venosum oder Wunden von Patienten handeln, die korpulent oder Diabetiker sind oder einer Strahlen- oder Chemotherapie unterzogen worden sind.

Synthetische Lipopeptidpräparate wären kostengünstig herstellbar und würden den natürlichen Ablauf der Wundheilung verstärken,. ohne prinzipiell in den komplizierten Regelmechanismus verschiedener Mediatoren der Wundheilung einzugreifen. Darüberhinaus würde eine Infektionsprophylaxe bewirkt, ohne daß Antibiotika oder andere, die Wundheilung z. B. schädigende Bakteriostatika eingesetzt werden müssten. Solche Lipopeptidpräparate wären auch bei Gesichtswunden einsetzbar, bei denen aus Gründen der Toxizität der üblichen Bakteriostatika bei Gefahr des Kontakts mit Augen oder dem Nasen- und Oralbereich Vorsicht geboten ist. Bei topischer Anwendung ist die Gefahr von systemischen Wirkungen wie Fieber so gut wie ausgeschlossen.

Nachstehend wird die Erfindung durch Abbildungen und Beispiele näher erläutert.

### Beispiel 1:

Als Beispiel der Wirksamkeit von synthetischen Lipopeptiden oder Liposomen, die in derartige Lipopeptide inkorporiert wurden, dient uns das Modell des Einströmens von Granulozyten und Makrophagen in den Peritonealraum der Maus. Es werden NMRI-Auszuchtmäuse als Versuchstiere verwendet, um genetische Besonderheiten auszuschließen.

Das racemische Lipopeptid MALP-2 wurde gemäß Mühlradt et al. in (6) synthetissiert, die Verbindungen R-MALP-2 = S-[2,3-bispalmitoyloxy-(2R)-propyl] cysteinyl-GNNDESNISFKEK bzw. S-MALP-2 = S-[2,3-bispalmitoyloxy-(2S)-propyl] cysteinyl-GNNDESNISFKEK nach Zitat 7. MALP-haltige Liposomen werden wie folgt konstruiert: Die in Chloroform bzw. Chloroform/Methaanol (1+1) gelösten Lipide (Phosphatidylglycerin, Phosphatidylserin, Cholesterol, NBD-PE, molares Verhältnis 1,08 : 1 : 0,25 : 0,005) werden mit dem in 2-Propano1/H₂O (1+1) gelösten MALP-2 zusammenpipettiert und mittels Rotationsverdampfer eingeeengt. Vollständige Trocknung des Lipidfilms erfolgt über Nacht unter der Sterilbank. Resuspension des getrockneten Lipidfilms in Octylglucosid (100 mM in 0,05 M Tris-gepufffertem NaCl, pH 7), 30 min. bei 37 °C im Wasserbad. Dialyse gegen das 50-fache Volumen NaCl (0,1 M Tris-gepuffert, pH 7) bei Raumtemperatur, 2 x Wechsel des Außendialysats nach jeweils 24 Stunden.

Die erhaltene Liposomensuspension wird insgesamt 3 x mit NaCl gewaschen (Zentrifugation 30 min. bei 47800 g, 4 °C) und im Anschluß daran in NaCl resuspenidert.

Zum Zeitpunkt 0 werden die folgenden Präparate steril und in physiologischer Kochsalzlösung in den Bauchraum der Versuchstiere injiziert. Gruppen von 6 Mäusen werden nach unterschiedlichen Zeiten getötet, der Bauchraum mit 1,2 ml steriler Kochsalzlösung gespült und die Leukozytenzahl und -zusammensetzung dieser Zellsuspension bestimmt.

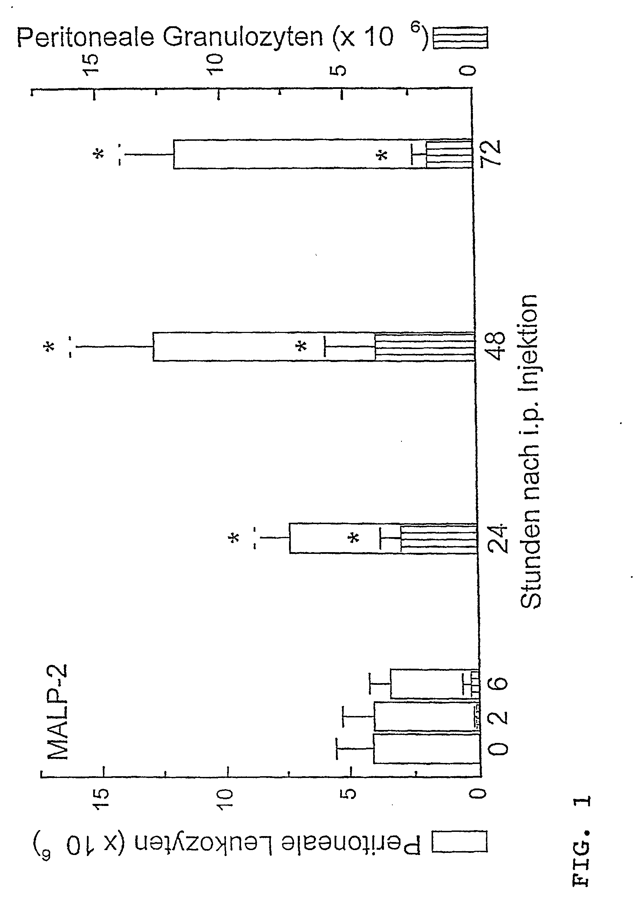

**Fig. 1** zeigt den Einstrom von Gesamtleukozyten bzw. Granulozyten als Reaktion auf die intraperitoneale Injektion von 9 µg racemischem MALP-2 in NMRI-Mäuse (Gruppen von 6 Tieren).

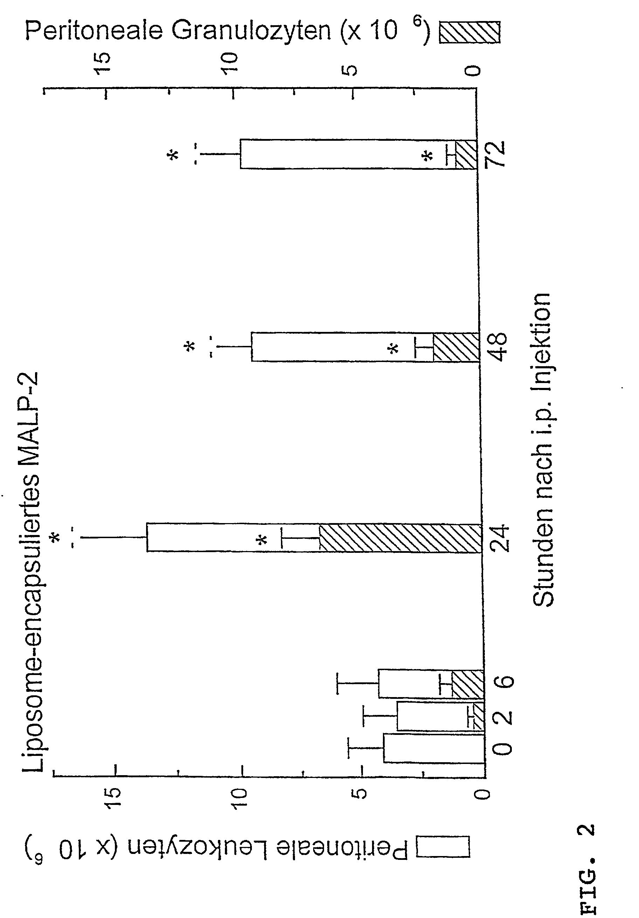

**Fig. 2** zeigt den Einstrom von Gesamtleukozyten bzw. Granulozyten als Reaktion auf die intraperitoneale Injektion von 0.2 mg Liposomen, die 9 µg MALP-2 enthielten, in NMRI-Mäuse (Gruppen von 6 Tieren).

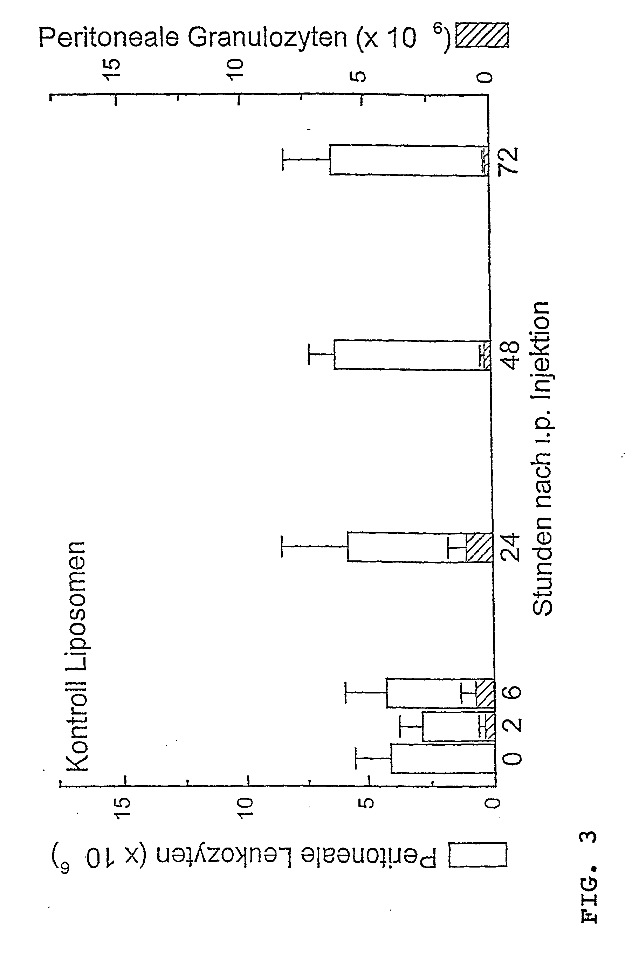

**Fig. 3** zeigt den Einstrom von Gesamtleukozyten bzw. Granulozyten als Reaktion auf die intraperitoneale Injektion von 0.2 mg Kontroll-Liposomen, die kein MALP-2 enthielten, in NMRI-Mäuse (Gruppen von 6 Tieren).
**Fig. 4A** zeigt die Infiltration von Leukozyten in die Rückenhaut einer NMRI Maus, 3 Tage nach intrakutaner Injektion von 2 µg R-MALP, das in Liposomen inkorporiert war.
**Fig. 4B** zeigt im Vergleich: Rückenhaut einer unbehandelten Maus.
**Fig. 5** zeigt den Bereich des nach MALP Injektion entstandenen neuen Gewebes mit neuen Gefäßen (Bildmitte).

In der **Tabelle 1** sind die wesentlichen Werte, d. h. der nach Injektion der Präparate erfolgende Anstieg an Granulozyten und der spätere Anstieg an Makrophagen zusammengefaßt. Es ergibt sich eine Zunahme gegenüber unbehandelten Kontrolltieren von Granulozyten um das etwa Hunderfache, während die Makrophagen nach 3 Tagen um das Doppelte bis Dreifache zugenommen haben.

Misst man 2 Std. nach Applikation der Mykoplasmen oder Lipopeptidpräparate die chemotaktisch wirksamen Chemokine MIP-1α, MIP-2 oder KC im Serum der Versuchstiere, so findet man signifikant erhöhte Aktivitäten.

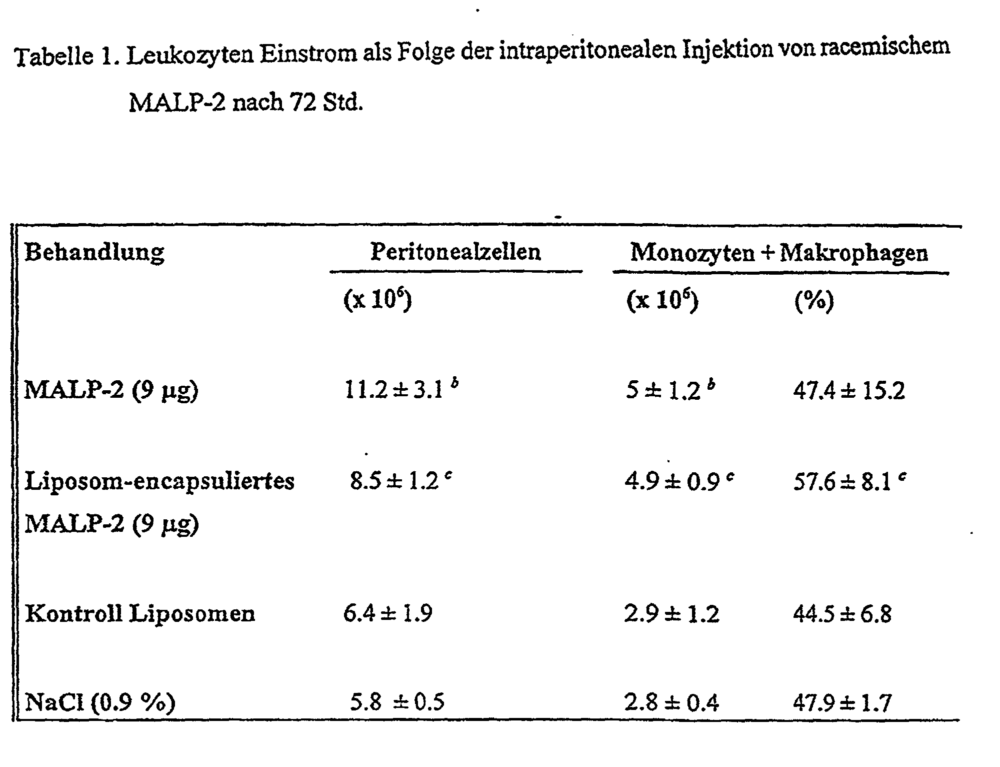

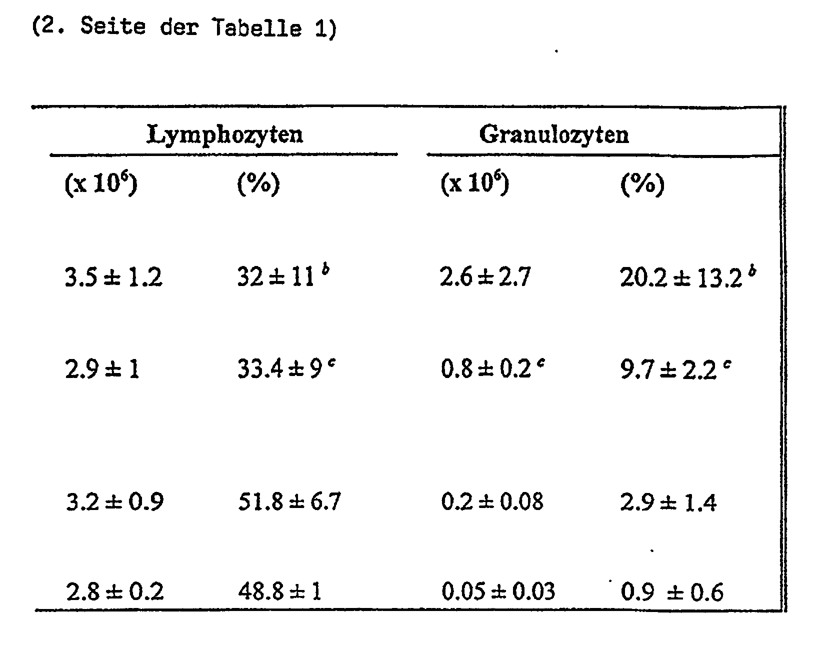

Es wurden Gruppen von 6 Tieren verwendet.
^{*b*} Signifikant unterschiedlich (P < 0.05) gegenüber Kochsalz behandelten Konrolltieren (nach Student's t-test)
^{*c*} Signifikant unterschiedlich (P < 0.05) gegenüber Tieren, die Kontroll Liposomen erhielten ( nach Student's t-test)

### Beispiel 2:

Auch durch Injektion von z. B. 15 µg Endotoxin Lipopolysaccharid aus Salmonella typhimurium kann Einströmen von Leukozyten erreicht werden, jedoch leiden die Versuchstiere unter dieser Behandlung. Dies ist nicht zuletzt auf einen meßbaren Anstieg an Tumor Nekrose Faktor-α (TNF) im Serum der Tiere zurückzuführen. Es wurde im Serum der Endotoxin-behandelten Tiere nach 1,5 Std. zwischen 2500 und 40000 pg/ml TNF gemessen (Kochsalz behandelte Kontrolltiere < 250 pg/ml). Dagegen wurde nach Anwendung der Mykoplasma-Präparate in dem oben genannten Beispiel 1 kein signifikanter Anstieg an TNF gegenüber Kontrolltieren gemessen.

### Beispiel 3:

Die Bedeutung des asymmetrischen C-Atoms an C2 der Dihydroxypropylgruppe zeigt die **Tabelle 2**. Hier wurden wie oben Gruppen von 5 NMRI-Mäusen unterschiedliche Mengen R-MALP-2 = S- [2,3-bispalmitoyloxy- (2R) -propyl] cysteinyl-GNNDESNISFKEK bzw. S-MALP-2 = S-[2,3-bispalmitoyloxy- (2S) -propyl] cysteinyl-GNNDESNISFKEK intraperitoneal appliziert und nach 3 Tagen die Gesamtzahl sowie Zusammensetzung der Peritonealleukozyten bestimmt. Das R-MALP-2 ist deutlich wirksamer.

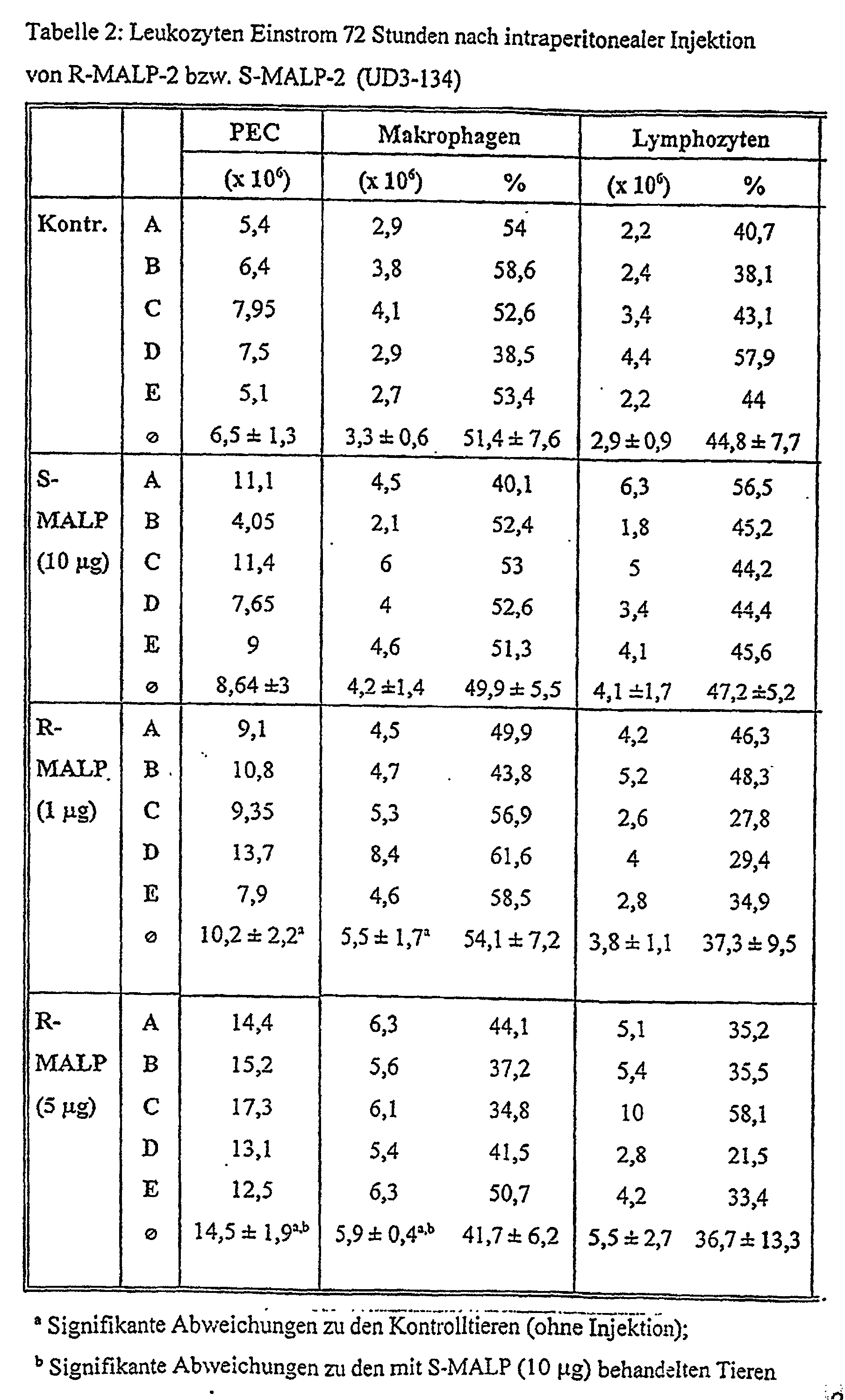

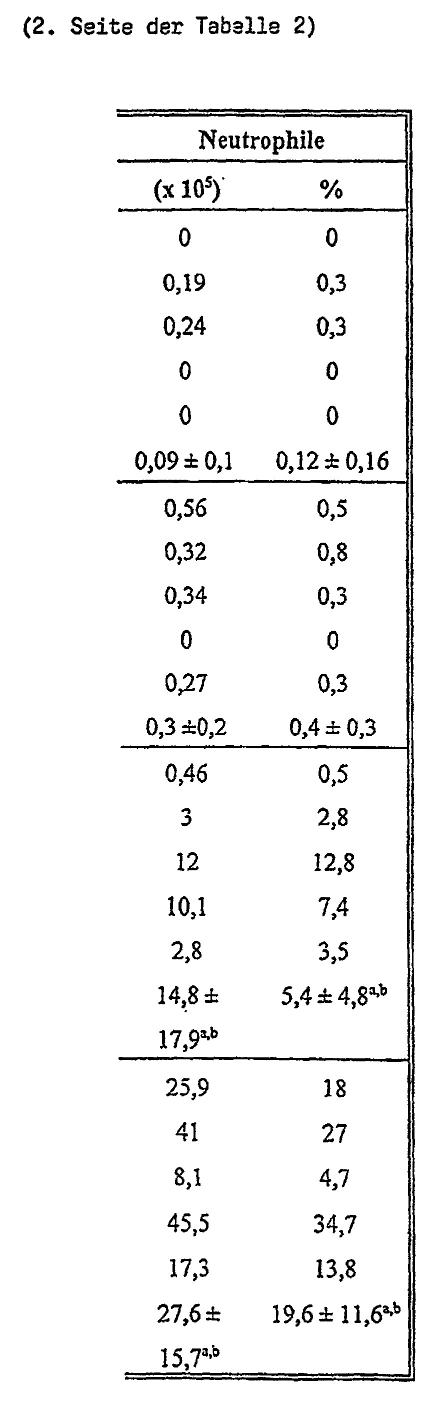

### Beispiel 4:

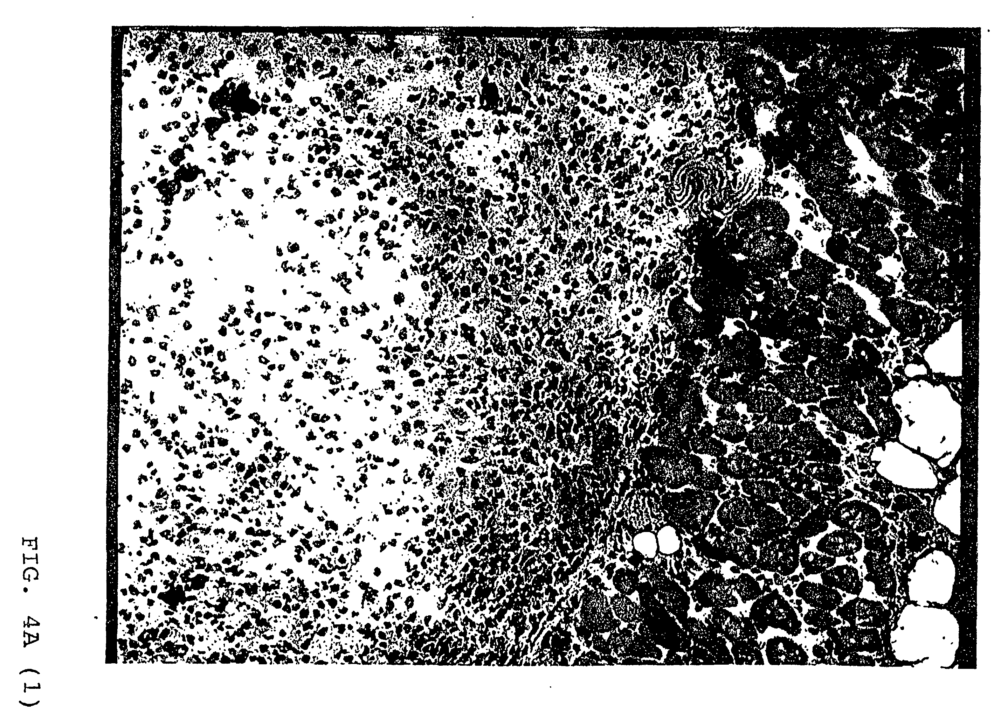

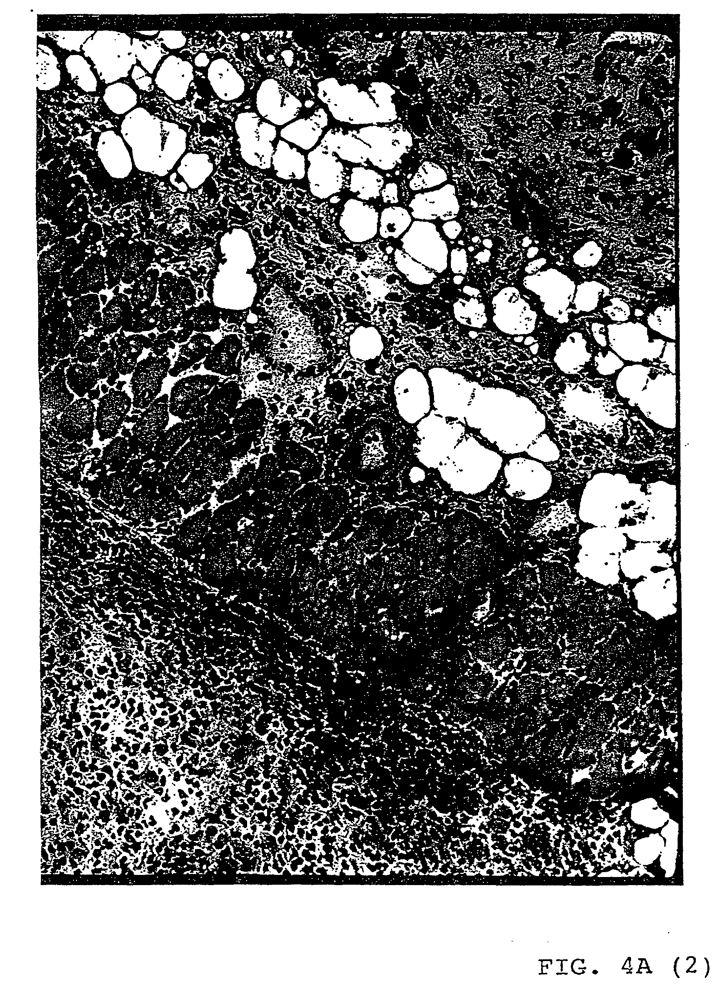

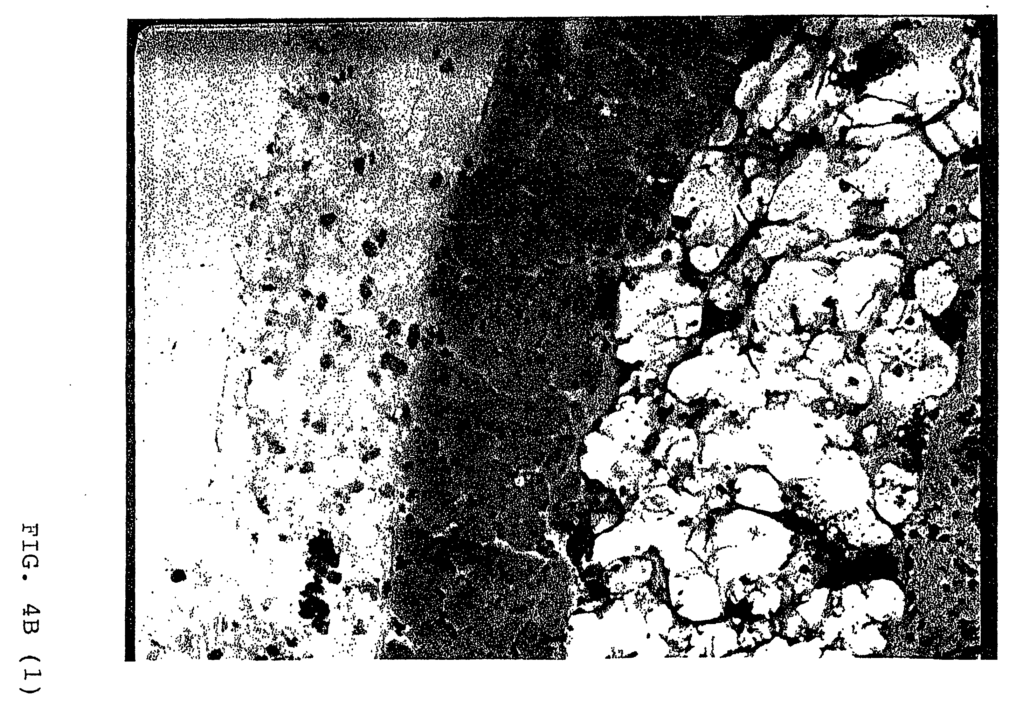

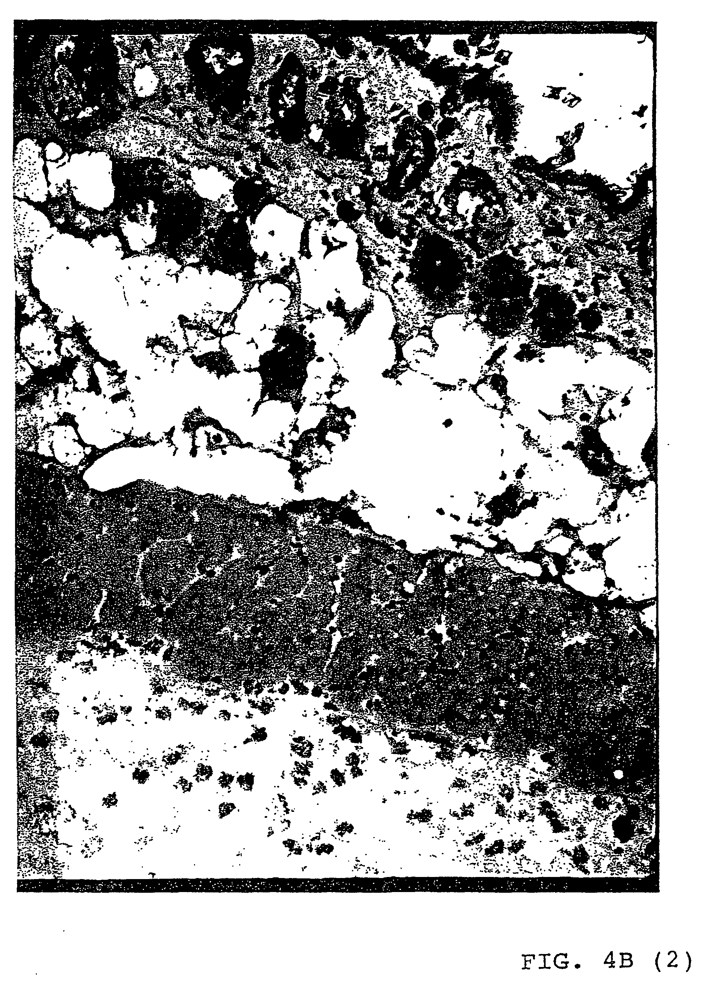

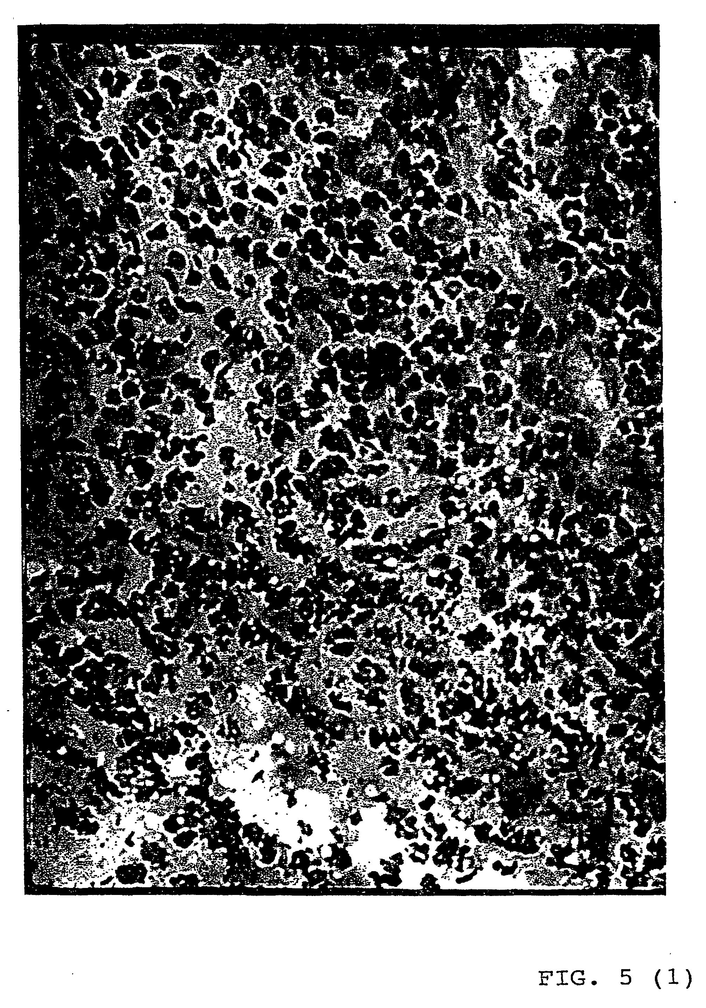

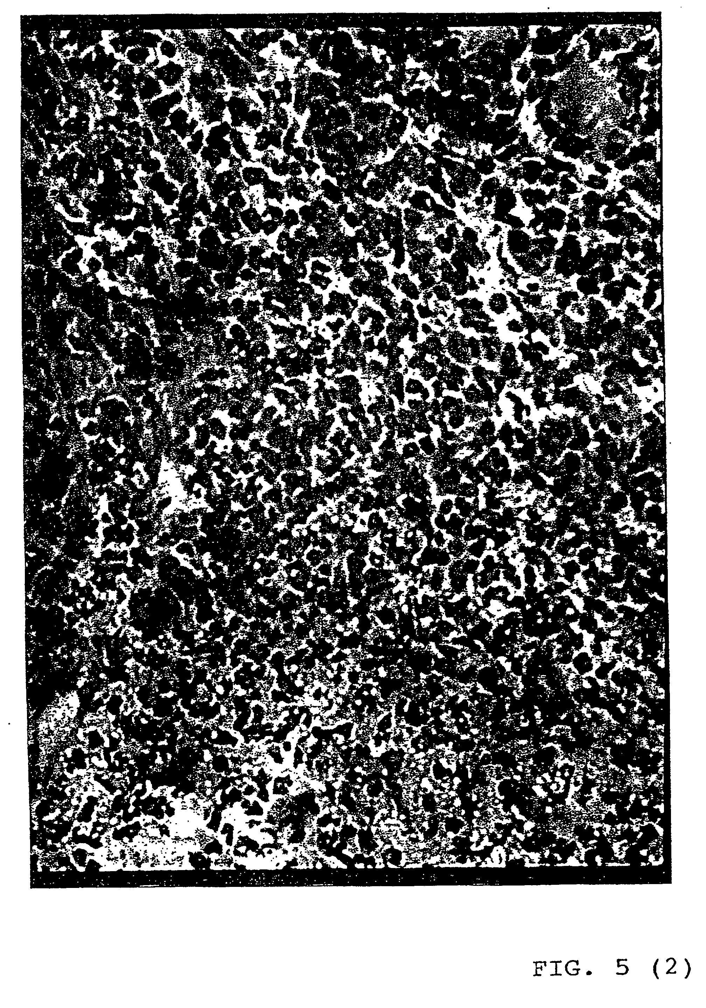

Injiziert man 2 µg freies R-MALP oder 2 µg R-MALP in 0,1 mg Liposomen inkorporiert intracutan in die Haut von NMRI-Mäusen, so bildet sich nach 3 Tagen an der Injektionsstelle eine deutliche Ansammlung von Leukozyten (siehe **Abb. 4A** und **4B**), bzw. nach 6 Tagen bilden sich neues Gewebe und Gefässe **(Abb. 5).** Dies zeigt, daß die Präparate in der Haut wirksam sind und in der Lage sind, die Wundheilung zu fördern.

### Referierter Stand der Technik

1. Quentmeier et al. in Infect. Immun., 58 (1990) 1273-1280,
2. Mühlradt & Frisch in Infect. Immun., 62 (1994) 3801-3807.
3. Mühlradt & Schade in Infect. Immun., 59 (1991) 3969-3974.
4. Metzger et al. in Int. J. Pep. Protein Res., 38 (1991) 545-554.
5. Metzger et al. in J. Pept. Sci., 3 (1995) 184-190.
6. Mühlradt et al. in J. Exp. Med., 185 (1997) 1951-1958
7. Metzger et al. in J. Medicinal Chem., 34 (1991) 1969-1974.

## Patentansprüche

1. Verwendung eines physiologisch verträglichen Lipopeptids oder Lipoproteins, das N-terminal eine Dihydroxypropyl-cystein-Gruppe mit 2 esterartig gebundenen, gegebenenfalls langkettigen Fettsäuren trägt, die gleich oder verschieden sind, zur Herstellung eines pharmazeutischen Präparats zur Tier- oder Human-Wundbehandlung.

2. Verwendung nach Anspruch 1, **dadurch gekennzeichnet, daß** das Lipopetid oder Lipoprotein aus einem Mykoplasma-Klon erhältlich ist.

3. Verwendung nach Anspruch 2, **dadurch gekennzeichnet, daß** das Lipopeptid oder Lipoprotein aus einem Mykoplasma fermentans-Klon erhältlich ist.

4. Verwendung nach einem der vorherigen Ansprüche, wobei das Lipopeptid oder Lipoprotein wasserlöslich oder amphoter ist.

5. Verwendung nach einem der vorhergehenden Ansprüche von
S-[2,3-Bispalmitoyloxy-(2RS)-propyl]-cysteinyl-GNNDESNISFKEK oder
S-[2,3-Bispalmitoyloxy-(2R)-propyl]-cysteinyl-GNNDESNISFKEK oder
S-[2,3-Bispalmitoyloxy-(2S)-propyl]-cysteinyl-GNNDESNISFKEK
als Lipopetdid.

6. Verwendung nach einem der vorherigen Ansprüche, wobei das Lipopeptid oder Lipoprotein in Form einer Lösung zur epikutanen Anwendung, einer Injektionslösung, einer Salbe, einer Lotion, einer wäßrigen Suspension, eines damit imprägnierten oder beschichteten Pflasters, in Liposomen verkapselt oder an biologisch abbaubare Trägerpolymere gekoppelt vorliegen kann.

7. Verwendung nach einem der vorherigen Ansprüche, wobei es sich bei den Wunden um Wunden nach Verletzungen oder chirurgischen Eingriffen, um chronisch infizierte Wunden, Brandwunden, chronische Ulcera oder Ulcus venosum oder Wunden von Patienten handelt, die korpulent oder Diabetiker sind oder einer Strahlen- oder Chemotherapie unterzogen worden sind.

## Claims

1. Use of a physiologically acceptable lipopeptide or lipoprotein, which is provided N-terminally with a dihydroxy-propyl-cystein group with two ester-like linked optionally long-chain fatty acids which may be identical or different, for the production of a preparation for wound treatment of animals or humans.

2. Use according to claim 1, **characterised in that** the lipopeptide or lipoprotein is obtainable from a Mycoplasma clone.

3. Use according to claim 2, **characterised in that** the lipopeptide or lipoprotein is obtainable from a Mycoplasma fermentans clone.

4. Use according to any of the preceding claims, wherein the lipopetide or lipoprotein is water-soluble or amphoteric.

5. Use according to any of the preceding claims of S-[2,3-Bispalmitoyloxy-(2RS)-propyl]-cysteinyl-GNNDESNISFKEK or S-[2,3-Bispalmitoyloxy-(2R)-propyl]-cysteinyl-GNNDESNISFKEK or S-[2,3-Bispalmitoyloxy-(2S)-propyl]-cysteinyl-GNNDESNISFKEK as lipopeptide.

6. Use according to any of the preceding claims, wherein the lipopeptide or lipoprotein can be provided in form of a solution for epicutaneous application, an injection solution, an ointment, a lotion, an aqueous suspension, a patch impregnated or coated therewith, encapsulated in liposomes or coupled to carrier polymers which are biologically degradable.

7. Use according to any of the preceding claims, wherein the wounds are wounds resulting from lesions or surgery, chronically infected wounds, burns, chronical ulcera or ulcus venosum or wounds of patients who are corpulent or diabetics or have been subjected to a radiation or chemotherapy.

## Revendications

1. Utilisation d'un lipopeptide ou d'une lipoprotéine physiologiquement acceptable portant, à son extrémité N-terminale, un groupe dihydroxypropylcystéine avec deux groupes d'acides gras, éventuellement en forme de chaîne longue, identiques ou différents, liés par des groupes ester, pour la fabrication d'une préparation pharmaceutique pour de traitement de plaies chez l'homme ou les animaux.

2. Utilisation selon la revendication 1, **caractérisée par le fait que** le lipopeptide ou la lipoprotéine est susceptible d'être obtenu(e) à partir d'un clone de mycoplasme.

3. Utilisation selon la revendication 2, **caractérisée par le fait que** le lipopeptide ou la lipoprotéine est susceptible d'être obtenu(e) à partir d'un clone de *Mycoplasma fermentans.*

4. Utilisation selon l'une des revendications précédentes, **caractérisée par le fait que** le lipopeptide ou la lipoprotéine sont hydrosolubles ou amphotères.

5. Utilisation selon l'une des revendications précédentes de
S-[2,3-bis-palmitoyloxy-(2RS)-propyl]-cystéinyl-GNNDESNISFKEK ou de
S-[2,3-bis-palmitoyloxy-(2R)-propyl]-cystéinyl-GNNDESNISFKEK ou de
S-[2,3-bis-palmitoyloxy-(2S)-propyl]-cystéinyl-GNNDESNISFKEK en'tant que lipopeptide.

6. Utilisation selon une des revendications précédentes, dans laquelle le lipopeptide ou la lipoprotéine se présentent sous forme d'une solution pour application épicutanée, d'une solution injectable, d'une pommade, d'une lotion, d'une suspension aqueuse, d'un, pansement imprégné ou revêtu d'une telle composition, ou sont encapsulés dans des liposomes ou couplés à des polymères porteurs biodégradables.

7. Utilisation selon une des revendications précédentes, où les plaies sont des plaies résultant de blessures ou d'interventions chirurgicales, des plaies à infection chronique, des plaies de brûlures, des ulcères chroniques ou d'*Ulcus venosum* ou des plaies de patients obèses ou diabétiques, ou encore de patients ayant subi une radiothérapie ou chimiothérapie.
